(19) Europäisches Patentamt
European Patent Office
Office européen des brevets

(11) Veröffentlichungsnummer: **0 135 837**
**A2**

(12) # EUROPÄISCHE PATENTANMELDUNG

(21) Anmeldenummer: **84110283.3**

(22) Anmeldetag: **29.08.84**

(51) Int. Cl.⁴: **C 07 D 519/00**, A 61 K 31/335 // (C07D519/00, 513:00, 498:00),(C07D519/00, 498:00, 498:00),(C07D519/00, 498:00, 471:00),(C07D519/00, 498:00, 487:00)

(30) Priorität: **01.09.83 CH 4802/83**

(43) Veröffentlichungstag der Anmeldung: **03.04.85 Patentblatt 85/14**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Traxler, Peter, Dr., Bündtenring 5, CH-4124 Schönenbuch (CH)**

(74) Vertreter: **Zumstein, Fritz sen., Dr. et al, Bräuhausstrasse 4, D-8000 München 2 (DE)**

(54) **Neue polycyclische Hydrazone, Verfahren zu ihrer Herstellung, diese enthaltende pharmazeutische Zusammensetzungen, und ihre Verwendung.**

(57) Neuartige Hydrazone abgeleitet von 3-Formylrifamycin S oder SV als Aldehydo-Komponente und einem bi- oder tricyclischen N-Aminopiperazin als Hydrazino-Komponente der Formel

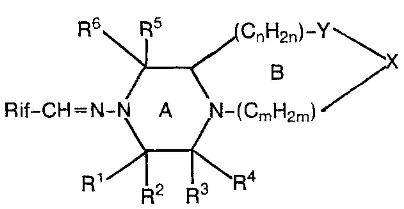

(I)

worin $R^1$, $R^2$, $R^3$, $R^4$, $R^5$ und $R^6$ unabhängig je ein Wasserstoffatom oder $C_{1-4}$-Alkyl,

m und n unabhängig je eine ganze Zahl von 0 bis 5,

X $C_{1-5}$-Alkyliden, Benzyliden oder $C_{1-4}$-Alkoxymethylen,

Y $C_{1-5}$-Alkyliden, $C_{1-4}$-Alkoxymethylen, Oxy, Thio oder gegebenenfalls substituiertes Imino der Formel -N(R)-, worin R für Wasserstoff, $C_{1-4}$-Alkyl, $C_{3-5}$-Alkenyl, $C_{3-12}$-Cycloalkyl oder Phenyl steht, oder

X und Y zusammen ein 1,2-Cycloalkylen oder o-Phenylen, welche durch 1-3 $C_{1-4}$-Alkylreste substituiert sein können, und

Rif einen durch die freie Valenz in 3-Stellung sich bindenden Rest von Rifamycin S bzw. SV bedeutet, zeichnen sich durch eine hohe und langdauernde antituberkulotische Wirksamkeit aus. Sie werden in konventioneller Weise, z.B. durch Kondensation ihrer Bildungskomponenten, hergestellt.

EP 0 135 837 A2

CIBA-GEIGY AG 4-14572/=

Basel (Schweiz)

Neue polycyclische Hydrazone, Verfahren zu ihrer Herstellung, diese enthaltende pharmazeutische Zusammensetzungen, und ihre Verwendung.

Gegenstand der Erfindung sind neue polycyclische Hydrazone abgeleitet von 3-Formylrifamycin S oder SV als Aldehydo-Komponente und einem bi-oder tricyclischen N-Aminopiperazin als Hydrazino-Komponente, insbesondere einem 1-Aminopiperazin mit einem 3,4-anellierten mono- oder bicyclischen Ringsystem. Speziell betrifft die Erfindung Hydrazone der Formel

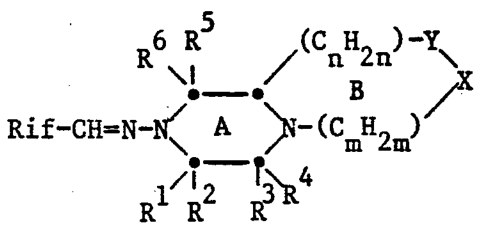

(I)

worin $R^1$, $R^2$, $R^3$, $R^4$, $R^5$ und $R^6$ unabhängig je ein Wasserstoffatom oder $C_{1-4}$-Alkyl,

m und n unabhängig je eine ganze Zahl von 0 bis 5,

X $C_{1-5}$-Alkyliden, Benzyliden oder $C_{1-4}$-Alkoxymethylen,

Y $C_{1-5}$-Alkyliden, $C_{1-4}$-Alkoxymethylen, Oxy, Thio oder gegebenenfalls substituiertes Imino der Formel -N(R)-, worin R für Wasserstoff, $C_{1-4}$-Alkyl, $C_{3-5}$-Alkenyl, $C_{3-12}$-Cycloalkyl oder Phenyl steht, oder

X und Y zusammen ein 1,2-Cycloalkylen oder o-Phenylen, welche durch 1-3 $C_{1-4}$-Alkylreste substituiert sein können, und

Rif einen durch die freie Valenz in 3-Stellung sich bindenen Rest von Rifamycin S (Chinon-Form) oder Rifamycin SV (Hydrochinon-Form) der Partialformeln

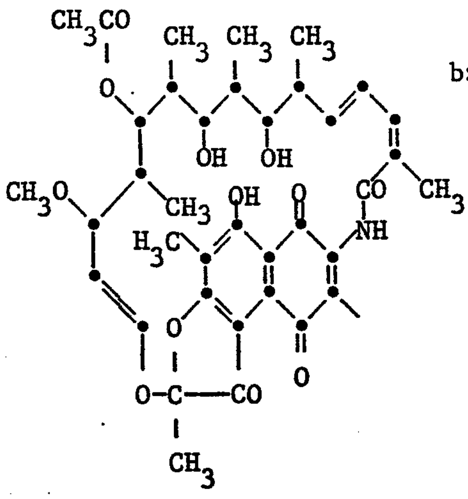

bzw.

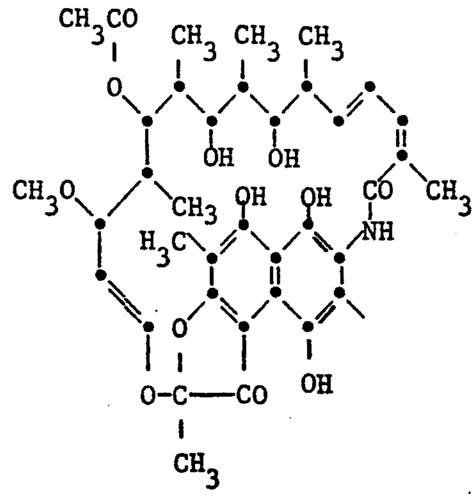

[Rif S] [Rif SV]

darstellt, sowie Salze davon.

Die Erfindung betrifft ferner auch Verfahren zur Herstellung von Verbindungen der Formel I (einschliesslich ihrer Salze), diese enthaltende pharmazeutische Zusammensetzungen und ihre Herstellung, sowie die Verwendung dieser Verbindungen und pharmazeutischen Präparate.

Infolge der sehr engen Beziehung zwischen der 1,4-Chinon- und -Hydrochinon-Form (entsprechend Rifamycin S und SV) und der Leichtigkeit, mit welcher die beiden Formen ineinander übergehen, sind überall, wo nicht spezifisch anders angegeben, beide Formen im Gegenstand der Erfindung inbegriffen.

Ein $C_{1-4}$-Alkyl kann Ethyl, Propyl und Butyl, ferner auch i-Propyl, i-Butyl, sec-Butyl und tert-Butyl, vorzugsweise aber Methyl sein. Entsprechende allgemeine und bevorzugte Bedeutungen hat auch der Alkylrest im $C_{1-4}$-Alkoxymethylen. Ein $C_{3-5}$-Alkenyl hat einen analogen Aufbau mit einer Doppelbindung, die sich vorzugsweise in 2- oder 3-Stellung befindet; hervorzuheben ist Allyl und Methallyl.

Ein $C_{1-5}$-Alkyliden ist beispielsweise Pentyliden, Butyliden, 1-, 2- oder 3-Methylbutyliden, 1-Ethylpropyliden, 1,2-Dimethylpropyliden, 1- oder 2-Methylpropyliden, und vorzugsweise Propyliden, Isopropyliden, Ethyliden und vor allem Methylen.

Die zweiwertigen Reste $-C_mH_{2m}-$ und $-C_nH_{2n}-$, welche den Piperazinring A mit Symbol X bzw. Y verbinden, stellen einen einfachen Valenzstrich (wenn m bzw. n für 0 steht), ein $C_{1-5}$-Alkyliden oder ein $C_{2-5}$--Alkylen dar. Das $C_{1-5}$-Alkyliden, welches den Piperazinring A von X bzw. Y durch 1 C-Atom trennt, hat eine der oben genannten allgemeinen oder bevorzugten Bedeutungen. Das $C_{2-5}$-Alkylen, welches den Piperazinring A von X bzw. Y durch 2-5 C-Atome trennt, leitet sich von einem verzweigten oder, vorzugsweise, geradkettigen Alkan ab, in welchem die beiden freien Valenzen von zwei verschiedenen, beliebig am Ende oder in der Mitte der Kette situierten C-Atomen ausgehen, und ist beispielsweise Pentamethylen, 1- oder 2-Methyltetramethylen, 1- oder 2-Ethyltrimethylen, 1,1-, 1,2-, 1,3- oder 2,2-Dimethyltrimethylen, Propylethylen, Isopropylethylen, 1-Ethyl-(1- oder 2)--methylethylen und 1,1,2-Trimethylethylen, ferner Tetramethylen, 1- oder 2-Methyltrimethylen, Ethylethylen und 1,1- oder 1,2-Dimethylethylen, sowie vorzugsweise Trimethylen, Propylen und Ethylen.
Ein $C_{3-12}$-Cycloalkyl ist ein einwertiger mono- oder bicyclischer Rest, dessen einzelne Ringe 3-8 Ringglieder aufweisen und mit einem bis vier $C_{1-4}$-Alkylresten, z.B. den oben charakterisierten, vorzugsweise Methylresten, substituiert sein können. Bevorzugt sind dabei symmetrische Kombinationen von Substituenten, d.h. solche, deren Anwesenheit die Bildung eines Asymmetriezentrums nicht zur Folge hat, wie insbesondere an einem und demselben C-Atom situierte (geminale) Paare gleicher Alkyle, wie sie z.B. in 4,4-Dimethylcyclohexyl oder 3,3,4,4-Tetramethylcyclopentyl vorkommen.
Ein 1,2-Cycloalkylen ist ein dem oben charakterisierten Cycloalkyl analoger zweiwertiger Rest, der jedoch vorzugsweise monocyclisch ist und 5-8 Ringglieder aufweist, wie beispielsweise 1,2-Cyclooctylen und 1,2-Cyclopentylen, sowie insbesondere 1,2-Cycloheptylen

und vor allem 1,2-Cyclohexylen; die beiden freien Valenzen sind zueinander cis- oder trans- orientiert und bilden mit Ring B die entsprechende Verknüpfung. Die Cycloalkylenreste können in analoger Weise wie die Cycloalkylreste substituiert sein, wie z.B. im 4,4-Dimethyl-1,2-cyclohexylen oder 3,3,5,5-Tetramethyl-1,2-cyclopentylen, unsubstituierte Reste sind aber bevorzugt. - Auch ein Phenyl oder o-Phenylen kann 1-4 oben charakterisierte $C_{1-4}$-Alkylreste, insbesondere Methylreste, tragen; vorzugsweise ist es aber unsubstituiert.

Der mit dem Piperazinring A anellierte Ring B besteht vorzugsweise aus insgesamt 5-8, insbesondere 5 oder 6 und vor allem aus 6 Ringgliedern.

Jedes der Symbole $R^1$ - $R^6$ einzeln bedeutet vorzugsweise Wasserstoff; wenn einige davon eine vom Wasserstoff unterschiedliche Bedeutung haben, dann sind es vorzugsweise höchstens 2 oder, ganz besonders, nur 1, wobei Methyl besonders bevorzugt ist. Eine vorteilhafte Anordnung bilden zwei gleiche Substituenten, welche geminal, d.h. an einem und demselben C-Atom, gebunden sind und somit kein zusätzliches Asymmetriezentrum (und, demzufolge, ein neues Diastereomerenpaar) verursachen.

Bevorzugte Verbindungen der Formel I sind z.B. diejenigen, worin m gleich 0 bis 3 und n gleich 1 bis 4 ist, wobei jeder der Reste $C_mH_{2m}$ und $C_nH_{2n}$ vorzugsweise geradkettig ist und/oder den Piperazinring A vom Symbol X bzw. Y durch höchstens 3, vorzugsweise höchstens 2 C-Atome trennt, höchtens einer der Symbole $R^1$-$R^6$ von Wasserstoff unterschiedlich ist und $C_{1-4}$-Alkyl, wie Methyl oder Ethyl, bedeutet, und eines der Symbole X und Y Ethyliden oder Methylen, während das andere $C_{1-4}$-Alkyliden, $C_{1-4}$-Alkoxymethylen, Oxy (-O-), Thio (-S-), Imino (-NH-) oder durch $C_{1-4}$ Alkyl substituiertes Imino darstellt oder X und Y zusammen für ein 1,2-Cycloalkylen mit 6-8 Ringatomen

oder o-Phenylen stehen.

Darunter sind ganz besonders bevorzugt z.B. Verbindungen der Formel I, worin m gleich 0 und n in einem vorzugsweise geradkettigen Rest $C_nH_{2n}$ gleich 1 bis 4 ist, höchstens eines der Symbole $R^1$-$R^6$ von Wasserstoff unterschiedlich ist, in welchem Falle es vorzugsweise Methyl bedeutet, und eines der Symbole X und Y Methylen, während das andere Ethyliden, Propyliden, $C_{1-4}$-Alkoxymethylen oder vorzugsweise Methylen darstellt, oder X und Y zusammen für 1,2-Cyclohexylen, 1,2-Cycloheptylen, 1,2-Cyclooctylen oder o-Phenylen stehen. Von diesen sind diejenigen hervorzuheben, worin m gleich 0 ist, $C_nH_{2n}$ Methylen, Trimethylen oder insbesondere Ethylen, $R^1$ Methyl oder insbesondere Wasserstoff, $R^2$-$R^6$ je Wasserstoff, X Ethyliden oder insbesondere Methylen und Y Propyliden, Isopropyliden, Methoxy- oder Ethoxy-methylen oder insbesondere Methylen darstellen oder X und Y zusammen für 1,2-Cyclohexylen oder o-Phenylen stehen.

Dementsprechend sind als die Hydrazin-Komponente insbesondere N-Aminopiperazine mit dem folgenden Grundgerüst, welches durch $C_{1-4}$-Alkyle und/oder $C_{1-4}$-Alkoxyle substituiert sein kann, besonders bevorzugt: 3-Amino-perhydro-pyrrolo[1,2-a]pyrazin, 3-Amino-perhydro-1H-pyrido-[1,2-a]pyrazin, 3-Amino-perhydro-pyrazino[1,2-a]azepin oder 3-Amino--perhydro-1H-pyrazino[1,2-a]azocin, bzw. 3-Amino-perhydro-pyrazino-[1,2-a]indol, 3-Amino-1,2,3,4,4a,5-hexahydro-pyrazino[1,2—a]indol, 3-Amino-perhydro-1H-pyrazino[1,2-a]chinolin, 3-Amino-2,3,4,4a,5,6-hexahydro-1H-pyrazino[1,2-a]chinolin.

Ganz besonders sind auch z.B. diejenigen Verbindungen der Formel I bevorzugt, worin je m und n unabhängig gleich 1 bis 3 ist, wobei die Reste $C_mH_{2m}$ und $C_nH_{2n}$ den Piperazinring A vom Symbol X bzw. Y jeweils höchstens durch 2, vorzugsweise durch 1 Kohlenstoffatom trennen, höchstens eines der Symbole $R^1$-$R^6$ von Wasserstoff unterschliedlich ist, in welchem Falle es vorzugsweise Methyl bedeutet,

und eines der Symbole X und Y Ethyliden oder Methylen, während das andere Oxy, Thio oder Imino darstellt, wobei das Imino vorzugsweise im oben angegebenen Sinne, insbesondere durch $C_{1-4}$-Alkyl, vor allem Isobutyl oder Methyl, substituiert sein kann. Von diesen sind diejenigen hervorzuheben, worin m und n jedes gleich 1 ist, $R^1$ Methyl oder vorzugsweise Wasserstoff, $R^2$-$R^6$ je Wasserstoff, X Methylen und Y Oxy, Thio, Imino oder $C_{1-4}$-Alkylimino, wie insbesondere Isobutylimino oder Methylimino, darstellt. Dementsprechend sind als die Hydrazin-Komponente insbesondere auch N-Aminopiperazine mit dem folgenden Grundgerüst, welches durch $C_{1-4}$-Alkyle C- und/oder N-substituiert sein kann, besonders bevorzugt:
6-Amino-perhydro-pyrazolo[2,1-c]-p-oxazin,
6-Amino-perhydro-pyrazolo[2,1-c]-p-thiazin, sowie
3-Amino-6-R-perhydro-1H-pyrazino[1,2-a]pyrazin, wobei R im letztgenannten Namen den Substituenten der Iminogruppe (einschliesslich Wasserstoff) bezeichnet.

Ganz besonders bevorzugt sind alle in den Beispielen genannten Verbindungen der Formel I in beiden Formen, d.h. sowohl der Chinon-(S-) wie auch der Hydrochinon-(SV-) -Reihe, und zwar auch dann, wenn nur eine Form ausdrücklich erwähnt wird, sowie entsprechende Salze.

Die meisten Verbindungen der Formel I können, ihrem individuellen Charakter nach, auch in Form von Salzen vorliegen. Diejenigen davon, welche eine genügende Acidität haben, wie insbesondere die Hydrochinone der SV-Reihe, können Salze mit Basen, wie insbesondere anorganischen Basen, vorzugsweise physiologisch verträgliche Alkalimetall-Salze , vor allem Natrium- und Kalium-Salze , bilden. Diejenigen der Verbindungen der Formel I, welche eine genügende Basizität haben, können als Säureadditionssalze, insbesondere als physiologisch verträgliche Salze, mit üblichen pharmazeutisch anwendbaren Säuren vorliegen; von der anorganischen Säuren sind die

Halogenwasserstoffsäuren, wie die Chlorwasserstoffsäure, sowie Schwefelsäure und Phosphor- bzw. Pyrophosphorsäure besonders zu nennen, von den organischen Säuren sind es in erster Linie die Sulfonsäuren, z.B. die aromatischen, wie Benzol- oder p-Toluol-sulfonsäure, Embonsäure und Sulfanilsäure, oder Niederalkansulfonsäuren, wie Methansulfon-, Ethansulfon-, Hydroxyethansulfon- sowie Ethylendisulfonsäure, oder aber auch aliphatische, alicyclische, aromatische oder heterocyclische Carbonsäuren, wie Ameisen-, Essig-, Propion-, Bernstein-, Glykol-, Milch-, Aepfel-, Wein-, Zitronen-, Fumar-, Malein-, Hydroxymalein-, Oxal-, Brenztrauben-, Phenylessig-, Benzoe-, p-Aminobenzoe-, Anthranil-, p-Hydroxybenzoe-, Salicyl- und p-Aminosalicylsäure, ferner Aminosäuren, insbesondere in der Natur vorkommende α-Aminosäuren, wie Glycin, Leucin, Methionin, Tryptophan, Lysin oder Arginin, sowie Ascorbinsäure. Verbindungen der Formel I, die sowohl basische wie saure funktionelle Gruppen enthalten, können auch als innere Salze vorliegen.

Einige Hydrazone abgeleitet von 3-Formylrifamycin SV sind bereits beschrieben worden. Insbesondere im U.S. 3'342'810 sind unter anderen antimikrobiell wirksamen Derivaten des letztgenannten Grundstoffes auch Hydrazone der Teilformel $=N-N(R_2)R_3$ beschrieben und beansprucht, worin $R_2$ und $R_3$, neben einer Reihe Bedeutungen für jedes Symbol individuell, beide gemeinsam und zusammen mit dem Stickstoffatom für ein heterocyclischer Ring mit 5-7 Atomen stehen. Ausser dieser Definition im Patentanspruch 6 ist keine nähere Charakterisierung des heterocyclischen Ringes in der Beschreibung zu finden, lediglich sind 9 Beispiele spezifischer Einzelverbindungen (einschliesslich biologischer Wirkungsdaten) in der Tabelle, Spalte 3, Verbindungen 3-14, angeführt.Von diesen wird im weiteren Text (Spalte 5, Zeilen 64-75, und Spalte 6, Zeilen 30-45) das Hydrazon von 3-Formylrifamycin SV mit 1-Amino-4-methylpiperazin wegen seiner hervorragenden antituberkulösen Wirksamkeit hervorgehoben und im spezifischen Anspruch 15 geschützt. Diese Verbindung gehört bis

jetzt, unter dem generischen Namen Rifampicin, zu den führenden Therapeutika zur Bekämpfung von Tuberkulose. Hydrazone mit mehr als einem heterocyclischen Ring wurden nicht erwogen oder angedeutet.

Obwohl Rifampicin zu den besten Mitteln für die Behandlung von Tuberkulose - Infektionen zählt, ist in manchen Fällen seine relativ kurze Verweildauer im Organismus ein wesentlicher Nachteil. Die Bereitstellung von Arzneimitteln, die in ihrer Wirkung gegen Tuberkulose-Infektion dem Rifampicin analog wären, aber eine verlängerte Wirkungsdauer aufweisen würden, ist daher eine der vordringlichsten Aufgaben auf diesem Gebiete. Bisher ist es nicht gelungen, auch unter andersartig modifizierten Rifamycin-Derivaten, welche zum Teil eine sehr gute antituberkulöse Wirkung entfalten (in Einzelfällen bis das Dreifache des Rifampicin), Wirkstoffe zu finden, die im bezug auf Langzeit-Wirkung dem Rifampicin ebenbürtig oder sogar überlegen wären.

Es wurde nun gefunden, dass sich die neuen Verbindungen gemäss der vorliegenden Erfindung sowohl durch eine gute antituberkulöse Wirksamkeit, welche, insbesondere in vivo, diejenige des Rifampicins übertrifft, als auch durch eine wesentlich erhöhte Verweildauer im Organismus auszeichnen, wie anhand der folgenden Datenzusammenstellung gezeigt werden kann.

Legende zur Tabelle I.

(1) Hydrazone des 3-Formylrifamycin SV; die Struktur wird charakterisiert durch das an den Piperazinring (A) anellierte Ringsystem (B); (1A) - der Piperazinring (A) trägt Methyl in 2-Stellung

(2) Testorganismus: Mycobacterium tuberculosis TB $H_3R_v$

(3) Minimale Hemmkonzentration (minimum inhibitory concentration) im Plattentest in vitro

(4) in vivo - Test (Maus); Verabreichung p.o.

(5) Halbwertszeit der Ausscheidung (in Stunden)

(6) Höchstkonzentration des Wirkstoffes in Blutplasma

(7) Vergleichssubstanz: Hydrazon des 3-Formylrifamycins SV mit 1-Amino-4-methylpiperazin.

0135837

Tabelle I:

| Bsp. Nr. | Wirkstoff (1) | Antituberkul. Wirkung (2) | | Pharmakokinetik | | | |
|---|---|---|---|---|---|---|---|
| | | | | Maus | | Ratte | |
| | | (3) MIC µg/ml | (4) $ED_{50}$ mg/kg | (5) t/2 h | (6) $C_{max}$ µg/ml | (5) t/2 h | (6) $C_{max}$ µg/ml |
| 1 | Piperidin | 0,03 | 0,8 | 20 | 27,3 | 17,0 | 24,5 |
| 2 | Pyrrolidin | 0,1 | 2,5 | 9,1 | 57,7 | 13,2 | 11,6 |
| 3 | 6-Methylpiperidin | 0,4 | 1 | 14 | 22,6 | | |
| 4 | 5-Ethylpiperidin | | | | | | |
| | Isomeres A | 0,01 | 0,8 | 55,1 | 29,5 | | |
| | B | 0,03 | 1 | 44,6 | 26,4 | | |
| 5 | Piperidin; (1A) | | | | | | |
| | Isomeres A | 0,2 | 3 | 69,3 | 15 | 58,5 | 19,7 |
| | B | 0,4 | 2 | 66 | 38,8 | 44,7 | 16,4 |
| | C | 0,06 | 3 | 15 | 23,7 | | |
| | D | 0,06 | 3 | 25,1 | 33,6 | | |
| 6 | Perhydrochinolin | | | | | | |
| | Isomeres A | 0,03 | | 33,4 | 56,3 | | |
| | B | 0,007 | | 40,9 | 34 | | |
| 7 | 1,2,3,4-Tetrahydro-chinolin | 0,06 | 4,5 | 32,2 | 24 | | |
| 8 | Thiomorpholin | 0,2 | 8 | 8,9 | 72 | | |
| 9 | N'-Methylpiperazin | 0,1 | | | | | |
| | Rifampicin (7) | 0,03 | 5,9 | 6 | 24,8 | 3,8 | 13,9 |

Die neuen Verbindungen der Formel I können in an sich bekannter Weise durch geläufige allgemeine Verfahren hergestellt werden, z.B. indem man

a) ein 3-Formylrifamycin der Formel

Rif-CH=O (II)

worin Rif die oben genannte Bedeutung hat, oder ein funktionelles Derivat davon, mit einem N-Aminopiperazin der Formel

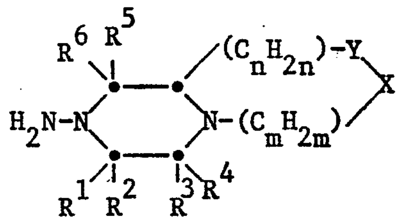

(III)

worin $R^1$-$R^6$, n, m, X und Y die oben angegebenen Bedeutungen haben, umsetzt, oder

b) zur direkten Herstellung von Derivaten der S-Reihe die 3-(Rifamycin S)-sulfonsäure der Formel

$[Rif\ S]-SO_3H$ (IV)

worin Rif die oben genannte Bedeutung hat, mit Formaldehyd-hydrazon eines N-Aminopiperazins der Formel

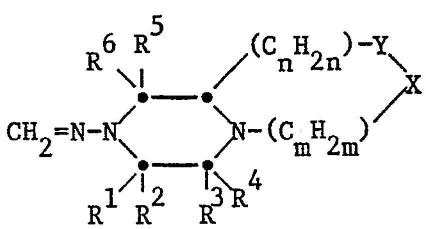

(V)

worin $R^1$-$R^6$, n, m, X und Y die oben angegebenen Bedeutungen haben, umsetzt, und,

gewünschtenfalls, wenn eine Verbindung der Formel I in der Chinon-Form erwünscht ist, eine in der Hydrochinon-Form vorliegende Verbindung der Formel I mit einem Oxidationsmittel behandelt, und/oder, wenn eine Verbindung der Formel I in der Hydrochinon-Form erwünscht ist, eine in der Chinon-Form vorliegende Verbindung der Formel I mit einem Reduktionsmittel behandelt und/oder eine in freier Form vorliegende Verbindung der Formel I in ein Salz davon überführt oder die Verbindung der Formel I aus einem Salz davon freisetzt.

Die Umsetzung eines 3-Formylrifamycins der Formel II oder eines reaktionsfähigen funktionellen Derivats davon, mit dem Hydrazin der Formel III erfolgt in an sich bekannter Weise, z.B. gemäss dem obengenannten U.S. Patent. Vornehmlich lässt man freies 3-Formylrifamycin SV und das N-Aminopiperazin (III) in etwa äquimolaren Mengen (oder einem kleinen Ueberschuss an diesem) und vorzugsweise in Anwesenheit eines organischen Lösungsmittels, wie eines Alkohols, z.B. Methanol, Ethanol oder Isopropylalkohol, eines offenkettigen oder cyclischen Ethers, z.B. Diethylether, 1,2-Dimethoxy- oder 1,2-Diethoxy-ethan, Tetrahydrofuran oder Dioxan, eines aliphatischen Esters oder Amids, z.B. Ethylacetat bzw. Dimethylformamid, ferner auch Dimethylsulfoxids und Acetonitrils, oder in einem Gemisch davon, bei Temperaturen von etwa -20° bis etwa 50°C, vorzugsweise zwischen Nullpunkt bis Raumtemperatur, reagieren. Die N-Aminopiperazin-Komponente (III) kann man auch in Form eines Säureadditionssalzes vorlegen und die Base erst in situ durch Zugabe eines basischen Hilfsstoffes, wie eines organischen Alkalimetallsalzes, z.B. Natrium- oder Kalium-acetat, oder einer tertiären organischen Base, wie eines tertiären Amins, z.B. Triethylamin, N-Methyl- oder N-ethyl-piperidin oder N-Methylmorpholin, oder aber einer heterocyclischen aromatischen Base vom Typ Pyridin und dessen Homologen oder Chinolin, freisetzen. - Auch die Aldehyd-Komponente der Formel II kann in Form eines funktionellen Derivats vorliegen, z.B. als ein Alkalimetallsalz der Hydrochinon-Form des Ausgangsstoffes (II), oder insbesondere als

ein reaktionsfähiges Derivat mit funktionell abgewandelter Aldehydgruppe, z.B. als eine Verbindung der Formel

Rif-CH=Z (IIA),

worin Rif die obengenannte Bedeutung hat und insbesondere [Rif S] ist und Z eine Oximino-, N-substituierte Imino-, unsubstituierte oder N-(mono- oder di)-substituierte Hydrazono-, oder Semicarbazono--Gruppe ist. Die Substituenten der Imino- und Hydrazono-Gruppe sind einwertige Kohlenwasserstoffreste je mit höchstens 8 C-Atomen, wie insbesondere Alkyl- oder Cycloalkyl-Reste mit höchstens 7 C-Atomen, Phenyl oder Benzyl, oder analoge zweiwertige Reste, welche zusammen mit dem Stickstoffatom einen gesättigten monocyclischen Heterocyclus mit 5-7 Ringgliedern bilden und gegebenenfalls einen zusätzlichen Heteroatom, wie Sauerstoff, Schwefel(II), Stickstoff oder einfach mit $C_{1-4}$-Alkyl substituierten Stickstoff im Ring enthalten. Bevorzugt sind solche Substituenten und ihre Kombinationen, welche leicht flüchtige Amine bzw. Hydrazine ergeben, insbesondere solche mit einem Siedepunkt, bei normalem oder vermindertem Druck, von höchstens 60°C, wobei Methyl besonderen Vorzug hat. - Bei Verwendung eines solchen Derivats der Aldehyd-Komponente(II) arbeitet man auch in an sich bekannter Weise analog der oben für freies Aldehyd geschilderten Methode. Wenn man dann die Komponente(II) als Salz einer Base einsetzt, ist es vorteilhaft, das Reaktionsgemisch auf neutrale Reaktion einzustellen, z.B. durch Vorlegen der anderen Komponente (des N-Aminopiperazins III) in Form eines Säureadditionssalzes, oder aber durch vorsichtige Zugabe einer Säure, wie einer Carbonsäure, z.B. Essigsäure. Wenn man die Aldehyd-Komponente (II) in Form eines Derivats mit funktionell abgewandelter Aldehydgruppe einsetzt, so kann man zweckmässig in einem grösseren Ueberschuss der basischen Komponente (III), welche dann zugleich als Lösungsmittel dient, arbeiten. Die Reaktionsbedingungen, vor allem Temperatur und Druck, werden dabei so eingestellt, dass die flüchtigen Reaktionsprodukte (z.B. das Amin bzw. Hydrazin der Formel $ZH_2$), die durch die Austauschreaktion

aus dem Ausgangsstoff (II) freigesetzt werden, kontinuierlich aus dem Reaktionsgemisch durch Abdestillieren entfernt werden. Zweckmässig wird der Druck so vermindert, dass die Temperatur beim Abdestillieren nicht etwa 60°C, vorzugsweise nicht etwa 40°C übersteigt. Unter ähnlichen Bedingungen kann man aber auch den Austausch in einem inerten organischen Lösungsmittel, wie einem der oben erwähnten, z.B. Dimethylsulfoxid, durchführen.

Die Verfahrensvariante b) wird auch in an sich bekannter Weise durchgeführt. Die Reaktionskomponente V wird dabei mit der 3-[Rifamycin S] -sulfonsäure (IV) vorzugsweise in einem Molarverhältnis von 1:1 bis 5:1, insbesondere von etwa 1:1 bis 2:1, und zweckmässig in einem inerten organischen Lösungsmittel, wie einem der oben erwähnten, vorzugsweise einen dipolaren Lösungsmittel, z.B. Acetonitril, Dimethylformamid, Hexamethylphosphortriamid oder vor allem Dimethylsulfoxid, zur Reaktion gebracht, wobei die Temperatur zwischen etwa 0° bis etwa 70°, vorzugsweise zwischen Raumtemperatur und etwa 50° liegt. Vornehmlich führt man die Reaktion in Anwesenheit eines Oxidationsmittels durch, welches zur Oxidation von Hydrochinonen zu Chinonen geeignet ist, wie insbesondere Mangandioxid, wobei man das Oxidationsmittel und die Hydrazonkomponente (V) in etwa äquimolarem Verhältnis einsetzt.

Die als Ausgangsstoff verwendeten Rifamycin-Derivate der Formeln II und IV sind bekannt. Auch Verbindungen der Formeln III und V sind entweder bekannt oder durch gewöhnliche Standardverfahren der synthetischen organischen Chemie zugänglich. So können die bi- oder tricyclischen N-Aminopiperazine der Formel III, z.B. durch Nitrosieren (z.B. mittels _in situ_ freigesetzter salpetriger Säure oder Stickstoffdioxid $N_2O_4$) des entsprechenden N-unsubstituierten bi- oder tricyclischen Piperazins der nachstehend definierten Formel VI und nachfolgende konventionelle Reduktion des gebildeten Nitrosamins z.B. mittels einem komplexen Hydrid, wie insbesondere Lithiumaluminiumhydrid, oder durch katalytische Hydrierung erhalten werden.

Bi- und tricyclische Piperazine der Formel

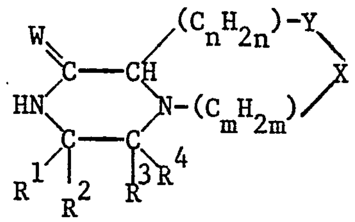

(VI)

worin $R^1$-$R^4$, m, n, X und Y die obgenannten allgemeinen oder bevorzugten Bedeutungen haben und W für $R^5$ und $R^6$ der obgenannten Definition steht, können ihrerseits z.B. durch eine einfache allgemein anwendbare Synthese erhalten werden, indem man einen mono- oder bicyclischen stickstoffhaltigen Heterocyclyl-2-carbonsäureester der Formel

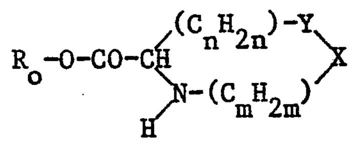

(VII)

worin $R_o$ eine $C_{1-4}$-Alkyl, insbesondere Methyl oder Ethyl, bedeutet und m, n, X und Y die obgenannten Bedeutungen haben, mit einem unsubstituierten oder substituierten Ethylenimin der Formel

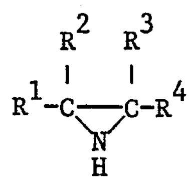

(VIII)

worin $R^1$-$R^4$ die obengenannten Bedeutungen haben, kondensiert und im erhaltenen Piperazon der Formel VI, worin $R^1$-$R^4$, m, n, X und Y die obengenannten Bedeutungen haben und W für Oxo steht, diese Oxogruppe in die Substituenten $R^5$ und $R^6$ in konventioneller Weise umwandelt. Diese Umwandlung erfolgt, wenn $R^5$ und $R^6$ je für Wasserstoff stehen, durch die Reduktion, z.B. mit Diboran oder einem komplexen Hydrid, wie insbesondere Lithiumaluminiumhydrid. Falls eines oder beide der Symbole $R^5$ und $R^6$ für Alkyl stehen, erfolgt die Umwandlung durch Behandeln mit einem entsprechenden organometallischen Reagens, wie Alkyllithium oder insbesondere einem Alkylmagnesiumhalogenid, z.B. -chlorid oder vorzugsweise -bromid, in geeignetem inerten

organischen Lösungsmittel. Bekanntlich können die Bedingungen so gelenkt werden, dass man eine "geminale Base" mit zwei Alkylsubstituenten, oder ein monoalkyliertes Produkt erhält; im letzteren Fall ist es notwendig, das primäre Zwischenprodukt der Formel VI, worin W ein Alkyl zusammen mit Hydroxyl bedeutet, noch zusätzlich, z.B. wie oben für die Oxogruppe angegeben wurde, weiterzureduzieren. Vornehmlich wird analog den in nachstehenden Beispielen angeführten Reaktionsbedingungen gearbeitet.

Ausgangsstoffe der Formel V werden aus den N-Aminopiperazinen der Formel III mit Formaldehyd unter allgemeinen Bedingungen erhalten, welche für die Bildung von Formaldehyd-Hydrazonen geläufig sind.

Im primären Reaktionsgemisch des erfindungsgemässen Verfahrens können beide Oxidationsstufen des Endstoffes, d.h. die 1,4-Chinonform der S-Reihe und die 1,4-Hydrochinon-Form der SV-Reihe, nebeneinander vorliegen. Zweckmässig isoliert man jedoch das gesamte Produkt in nur einer einzigen der beiden Formen, z.B. in der Hydrochinonform. Zweckmässig wird dieses Gemisch, wie unten noch näher beschrieben, vereinheitlicht, indem mittels Reduktion nur die Hydrochinonform (Derivat von Rifamycin SV) oder mittels Oxydation nur die Chinonform (Derivat von Rifamycin S) gebildet wird.

Die gewünschtenfalls durchzuführende erfindungsgemässe Umwandlung eines verfahrensgemäss erhältlichen Chinons der Formel I [Rif S] in das entsprechende Hydrochinon [Rif SV] bzw. eines verfahrensgemäss erhältlichen Hydrochinons der Formel I [Rif SV] in ein Chinon [Rif S], oder die Vereinheitlichung eines Gemisches der beiden Verbindungstypen erfolgt mittels Reduktion bzw. Oxidation. Diese Umwandlung kann an einem bereits isolierten Produkt oder, oft vorteilhafterweise, noch vor der Isolierung des gewünschten Produkts durchgeführt werden. Die Reduktion kann durch Behandeln mit einem, insbesondere einem zur Reduktion eines Chinons in das entsprechende Hydrochinon, geeigneten, Reduktionsmittels, wie einem Alkalimetall-,

z.B. Natrium-, -dithionit oder -hydrosulfit, Zink und Essigsäure, oder vorzugsweise mit Ascorbinsäure, die Oxidation durch Behandeln mit einem, insbesondere einem für die Umwandlung eines Hydrochinons in das entsprechende Chinon geeigneten, Oxidationsmittel, wie Luftsauerstoff, Wasserstoffperoxid, Alkalimetall-, z.B. Kalium-, -ferricyanid, einem Persulfatsalz, z.B. Ammoniumpersulfat, oder Mangandioxid, bewirkt werden, wobei die Oxidation vorzugsweise unter basischen Bedingungen durchgeführt wird. Die Chinone sind meist violettrot gefärbte Verbindungen, während die Hydrochinone üblicherweise gelbgefärbt sind und über besseres Kristallisationsvermögen verfügen.

Die gewünschtenfalls durchzuführende Salzbildung und Freisetzen der Grundformen der Verbindungen der Formel I aus ihren Salzen erfolgt auch in an sich allgemein bekannter, konventioneller Weise. - So werden Hydrochinone in die entsprechende Alkalimetallsalze durch Behandeln mit einer entsprechenden alkalisch reagierenden Verbindung, insbesondere Hydroxid, Carbonat oder Bicarbonat, überführt; die Salze können in freie Hydrochinon-Verbindungen durch Ansäuern, z.B. mit anorganischen Säuren, wie insbesondere Halogenwasserstoffsäuren, umgewandelt werden. - Basisch reagierende Endstoffe der Formel I können in ihre Säureadditionssalze z.B. durch Behandeln mit einer zur Salzbildung geeingeter Säure, wie einer der oben genannten umgewandelt werden; umgekehrt wird durch Behandeln mit basisch reagierenden Mitteln, wie mit anorganischen Hydroxiden, Carbonaten und Bicarbonaten, oder organischen Basen und Ionenaustauschern, eine solche basische Grundform einer Verbindung der Formel I freigesetzt. Innere Salze werden z.B. durch übliches acido-basisches Titrieren zum Neutralpunkt bzw. zum isoelektrischen Punkt gebildet.

Die Säureadditionssalze der neuen Verbindungen, wie z.B. die Pikrate, können auch zur Reinigung der erhaltenen Verbindungen dienen, indem man die freien Verbindungen in Salze überführt, diese abtrennt und aus den Salzen wiederum die freien Verbindungen gewinnt. Infolge der engen Beziehung zwischen den Verbindungen in freier Form und in

Form ihrer Salze sind im vorausgegangenen und nachfolgend unter den freien Verbindungen sinn- und zweckgemäss gegebenenfalls auch die entsprechenden Salze zu verstehen.

Die oben beschriebenen Endstoffe der Formel I, Ausgangsstoffe (III) und (V), sowie Zwischenprodukte werden gegebenenfalls als Razemate (d.h. Gemische von 2 Antipoden), Razematgemische (d.h. Gemische von 2 diastereomeren Razematen) und Diastereomerengemische (d.h. Gemische von 2 solchen Diastereomeren, die zueinander nicht als Antipoden stehen) erhalten und können in an sich bekannter Weise in individuelle Antipoden aufgetrennt werden.

Ein Razematgemisch resultiert insbesondere durch Umsetzung einer chiralen Reaktionskomponente in razemischer Form mit einer anderen chiralen Reaktionskomponente, die auch in razemischer Form vorliegt. Wenn eine der Reaktionskomponenten in razemischer Form, die andere als einzelner Antipode eingesetzt wird, wie es vorwiegend beim erfindungsgemässen Verfahren der Fall ist, resultiert ein Diastereomerengemisch. Diese Gemische beider Art sind durch physikalische Methoden trennbar, wobei im ersteren Fall 2 Razemate, im letzteren Fall 2 individuelle, nicht zueinander gehörende Diastereomere isoliert werden. Unter den Trennmethoden ist in erster Linie die fraktionierte Kristallisation zu erwähnen, weiter auch verschiedene Varianten der Adsorptions- und Verteilungschromatographie, bei leichtflüchtigen Gemischen auch die fraktionierte Destillation und insbesondere Gaschromatographie.

Wird bei den oben geschilderten Umsetzungen wie besonders bei der Herstellung von Ausgangsstoffen (III) und (V) der Fall ist, ein razemischer Ausgangsstoff mit einer achiralen Reaktionskomponente umgesetzt, so resultiert das Reaktionsprodukt wiederum als Razemat, welches man bekanntlich in individuelle Antipoden erst unter Herbeiziehen von chiralen Hilfsstoffen oder Hilfsmitteln nach an sich bekannten Methoden spalten kann. - Eine bevorzugte Methode besteht darin, dass man eine razemische freie Base mit einer optisch aktiven Säure zu einem Diastereomerengemisch zweier Säureadditionssalze umwandelt, dieses durch geeignete physikalische Methoden auftrennt, und jeden individuellen Diastereomeren separat in seine Bildungskomponenten, d.h. die Säure und den individuellen Antipoden der Base, in an sich bekannter Weise, z.B. durch Behandeln mit einer stärkeren Säure oder Base, umwandelt. - Als optisch aktive Säuren eignen sich z.B. optisch aktive Aminosäuren, insbesondere die in der Natur vorkommenden α-Aminosäuren der L-Reihe und ihre N-acylierten Derivate, die optisch aktiven Weinsäuren, insbesondere die d-Weinsäure, und ihre Derivate mit veresterten Hydroxylgruppen, d- und l-Mandelsäure, d-Camphercarbonsäure sowie d- oder l-Campher-10-sulfonsäure.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen.

Im Hinblick auf die oben beschriebenen pharmakologischen Eigenschaften der neuen Verbindungen umfasst die vorliegende Erfindung auch die Verwendung der erfindungsgemässen Wirkstoffe, allein, z.B. zusammen mit Hilfsstoffen, oder in Kombination mit anderen Wirkstoffen, insbesondere Antibiotika oder Chemotherapeutika, als Mittel zur Behandlung von Infektionen, insbesondere solchen, die durch Tuberkelbazillen, sowie durch Bakterien, insbesondere Kokken,

wie den genannten, hervorgerufen werden, und zwar sowohl als Heilmittel, wie auch als Desinfektionsmittel. Bei der Verwendung als Heilmittel werden die erfindungsgemässen Wirkstoffe vorzugsweise in Form von pharmazeutischen Präparaten zusammen mit konventionellen pharmazeutischen Trägermaterialien oder Hilfsstoffen verabreicht. Dabei werden z.B. an Warmblüter mit einem Körpergewicht von etwa 70 kg je nach Species, Körpergewicht, Alter und individuellem Zustand, sowie je nach Applikationsweise und insbesondere auch je nach der jeweiligen Empfindlichkeit des Krankheitserregers, tägliche Dosen von etwa 50 bis etwa 1000 mg verabreicht.

Die Erfindung betrifft im weiteren pharmazeutische Präparate, welche die Verbindungen der vorliegenden Erfindung als Wirkstoffe enthalten, sowie Verfahren zu ihrer Herstellung.

Bei den erfindungsgemässen pharmazeutischen Präparaten handelt es sich z.B. um solche zur enteralen, wie peroralen oder rektalen, sowie zur parenteralen Verabreichung an Warmblüter. Entsprechende Dosiseinheitsformen,insbesondere zur peroralen Verabreichung, z.B. Dragées, Tabletten oder Kapseln, enthalten vorzugsweise von etwa 50 bis etwa 500 mg, insbesondere etwa 100 bis etwa 300 mg des Wirkstoffes zusammen mit pharmazeutisch verwendbaren Träger- oder Hilfsstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier-und Schmiermittel, z.B. Kieselsäure, Talk, Stearinsäure oder Salze davon, wie Magnesium- oder Calciumstearat, und/oder Polyethylenglykol. Dragée-Kerne können mit geeigneten, gegebenenfalls Magensaftresistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten organischen Lösungsmitteln oder Lösungsmittelgemischen, oder, zur Herstellung von Magensaft-resistenen Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässerige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben, Schmelzpunkte sind nicht korrigiert. In der Dünnschichtchromatographie verwendet man Kieselgel als Adsorbens, die Lösungsmittelgemisch werden in Volum-Verhältnissen angegeben.

Beispiel 1: Hydrazon des 3-Formylrifamycin SV mit 3-Amino-perhydro-1H-pyrido[1,2-a]pyrazin.

Eine Lösung von 5 g 3-Formylrifamycin SV in 100 ml Tetrahydrofuran wird mit einer Lösung von 1,6 g 3-Amino-perhydro-1H-pyrido[1,2-a]pyrazin in 10 ml Tetrahydrofuran versetzt und bei Raumtemperatur während 6 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Kristallisation aus Ethylacetat orangerote Kristalle der Titelverbindung ergibt, welche unter Zersetzung unscharf schmelzen. Dünnschichtchromatographie mit Methylenchlorid-Methanol (9:1): Rf = 0,6; Massenspektrum (CDI/$CH_4$, negativ): $M^{-\bullet}$ = 862 (Molekulargewicht berechnet für $C_{47}H_{62}N_4O_{12}$ = 862).

A. Natriumsalz der Titelverbindung wird erhalten, indem man 3g des obigen freien Hydrazons in 10 ml Dioxan löst, mit einer Lösung von 0,929 g Natriumbicarbonat in 10 ml Wasser versetzt und die resultierende Lösung lyophilisiert.

B. Oxidation zum Chinon:

Eine Lösung von 2 g Hydrazon des 3-Formylrifamycin SV mit 3-Amino-perhydro-1H-pyrido[1,2-a]pyrazin (hergestellt wie oben beschrieben) in 50 ml Methylenchlorid wird während 2 Minuten mit pulverigem Mangandioxid bei Raumtemperatur intensiv gerührt, die festen Anteile abfiltriert und das Filtrat zur Trockne eingedampft. Es resultierten 2 g Hydrazon des 3-Formylrifamycin S mit 3-Amino-perhydro-1H-pyrido[1,2-a]pyrazin als amorpher blau-schwarzer Feststoff ohne scharfen Schmelzpunkt. Molekulargewicht gemäss Massenspektrum ($MH^+$=861) entspricht der Theorie für $C_{46}H_{60}N_4O_{12}$.

Das als Ausgangsmaterial verwendete 3-Amino-perhydro-1H-pyrido-[1,2-a]pyrazin kann folgendermassen hergestellt werden:

a) Nitrosierung:

Eine wässrige Lösung von 5,86 g Perhydro-1H-pyrido[1,2-a]pyrazin, vgl. M.E. Freed und A.R. Day: J. Org. Chem. 25, 2108 (1960), wird bei 5°C mit konzentrierter Salzsäure auf pH 1,1 gebracht und unter Kühlung auf 0-5°C mit einer Lösung von 3,18 g Natriumnitrit in 5 ml Wasser tropfenweise behandelt, weitere 2 Stunden bei 5-10°C gerührt und mit etwa 58 ml wässriger 2N-Lösung von Natronlauge auf pH 13 gestellt. Das Reaktionsgemisch wird mit 3 Portionen von jeweils 80 ml Ethylacetat extrahiert, und die vereinigten organischen Lösungen werden mit Natriumsulfat getrocknet und eingedampft. Der ölige Rückstand (6,76 g) wird ohne Reinigung für die nächste Stufe verwendet.

b) Reduktion:

Zu einer gerührten Suspension von 1,71 g Lithiumaluminiumhydrid in 150 ml Tetrahydrofuran wird bei Rückflusstemperatur eine Lösung von 6,76 g rohem 3-Nitroso-perhydro-1H-pyrido[1,2-a]pyrazin (hergestellt gemäss Stufe a) in 30 ml Tetrahydrofuran zugetropft. Das Reaktionsgemisch wird weitere 3 Stunden bei Raumtemperatur gerührt, unter Eiskühlung mit 10 ml wässriger 2N-Natronlauge-Lösung und nachher mit 10 ml Wasser versetzt und weitere 2 Stunden bei Raumtemperatur gerührt. Der Niederschlag wird durch Abnutschen entfernt und auf der Nutsche mit heissem Isopropylalkohol nachgewaschen. Das Filtrat (samt der Isopropylalkohol-Anteilen), im Vakuum eingedampft, ergibt das gewünschte 3-Amino-perhydro-1H-pyrido[1,2-a]pyrazin (6,68 g) als leicht gelb gefärbtes Oel in einer für die oben beschriebene Hydrazon-Bildung ausreichenden Reinheit.

Beispiel 2: Hydrazon des 3-Formylrifamycin SV mit 3-Amino-perhydro-pyrrolo[1,2-a]pyrazin.

Eine Lösung von 2 g 3-Formylrifamycin SV in 200 ml Tetrahydrofuran wird mit einer Lösung von 1,94 g 3-Amino-perhydro-pyrrolo[1,2-a] pyrazin in 10 ml Tetrahydrofuran versetzt und bei Raumtemperatur während 10 Minuten gerührt, bis gemäss Dünnschichtchromatographie alles 3-Formylrifamycin verbraucht ist. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Kristallisation aus Ether orangerote Kristalle von der Titelverbindung, Smp. $>$ 210°C unter langsamer Zersetzung; Dünnschichtchromatographie mit Methylenchlorid-Methanol (9:1): Rf= 0,55; ergibt. Massenspektrum ($DCl/CH_4$, negativ):$M^{-\bullet}$ = 848 (Molekulargewicht berechnet für $C_{45}H_{60}N_4O_{12}$ = 848).

Zur Herstellung von als Ausgangsstoff verwendeten 3-Amino-perhydro-pyrrolo[1,2-a]pyrazin kann man beispielsweise Perhydro-pyrrolo-[1,2-a]pyrazin, vgl. M.E.Freed und A.R.Day: J.Org.Chem. 25, 2108 (1960= und R.L.Peck und A.R.Day: J. Heterocyclic Chem. 6, 181 (1963) gemäss Beispiel 1, Präparation a) und b) nacheinander nitrosieren und reduzieren.

Beispiel 3: Hydrazon des 3-Formylrifamycin SV mit 3-Amino-8-methyl-perhydro-1H-pyrido[1,2-a]pyrazin.

Eine Lösung von 2 g 3-Formylrifamycin SV in 100 ml Tetrahydrofuran wird mit einer Lösung von 1,86 g 3-Amino-8-methyl-perhydro-1H-pyrido [1,2-a]pyrazin in 10 ml Tetrahydrofuran versetzt und bei Raumtemperatur während 10 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch

Chromatographie über Silicagel in 2 individuelle Stereoisomere aufgetrennt wird; diese werden durch Kristallisation aus Aceton-Ether-Hexan weiter gereinigt.
Beide Isomeren weisen analoge Massenspektra: (FAB, positiv) auf: $MH^+$ = 877 (Molekulargewicht bereichnet für $C_{47}H_{64}N_4O_{12}$ = 867).

Isomer A: Smp. 183-192°C (Zersetzung); Dünnschichtchromatographie mit Methylenchlorid-Methanol (9:1): Rf = 0,58
Isomer B: Smp. 166-190°C (langsame Zersetzung); Dünnschichtchromatographie mit Methylenchlorid-Methanol (9:1): Rf = 0,55.

Zur Herstellung vom als Ausgangsstoff verwendeten 3-Amino-8-methyl-perhydro-1H-pyrido[1,2-a[pyrazin kann man beispielsweise 8-Methyl-perhydro-1H-pyrido[1,2-a]pyrazin gemäss Beispiel 1, Präparation a) und b) nacheinander nitrosieren und reduzieren. Die letztgenannte Verbindung ist aus 2-Methyl-6-piperidincarbonsäure-ethylester gemäss dem Verfahren von Freed and Day, J.Org.Chem. 25, 2108 (1960) erhältlich.

Beispiel 4: Hydrazon des 3-Formylrifamycin SV mit 3-Amino-7-ethyl-perhydro-1H-pyrido[1,2-a]pyrazin.

Eine Lösung von 3 g 3-Formylrifamycin SV in 100 ml Tetrahydrofuran wird mit einer Lösung von 1,41 g 3-Amino-7-ethyl-perhydro-1H-pyrido-[1,2-a]pyrazin in 10 ml Tetrahydrofuran versetzt und bei Raumtemperatur während 20 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Chromatographie über 200 g Silicagel in 2 amorphe Stereoisomere der Titelverbindung aufgetrennt wird. Beide Isomeren ergeben analoges

Massenspektrum (FAB, negativ): $(M - H)^-$ = 889 (Molekulargewicht berechnet für $C_{48}H_{66}N_4O_{12}$ = 890).

Isomeres A: Smp. 173-177°C (Zersetzung); Dünnschichtchromatographie mit Ethylacetat-Cyclohexan (2:1): Rf = 0,19

Isomeres B: Smp. 173-180°C (Zersetzung); Dünnschichtchromatographie mit Ethylacetat-Cyclohexan 2:1): Rf = 0,31.

Zur Herstellung vom als Ausgangsstoff verwendeten 3-Amino-7-ethyl-perhydro-1H-pyrido[1,2-a]pyrazin kann man beispielsweise 7-Ethyl-perhydro-1H-pyrido[1,2-a]pyrazin gemäss Beispiel 1, Präparation a) und b) nacheinander nitrosieren und reduzieren. Die letztgenannte Verbindung ist aus 3-Ethyl-6-piperidincarbonsäure-ethylester gemäss dem Verfahren von Freed and Day, J.Org.Chem. 25, 2108 (1960) erhältlich.

Beispiel 5: Hydrazon des 3-Formylrifamycin SV mit 3-Amino-2-methyl-perhydro-1H-pyrido[1,2-a]pyrazin.

Eine Lösung von 20 g 3-Formylrifamycin SV in 500 ml Tetrahydrofuran wird mit einer Lösung von 5,8 g 3-Amino-2-methyl-perhydro-1H-pyrido[1,2-a]pyrazin in 20 ml Tetrahydrofuran versetzt und bei Raumtemperatur während 10 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches mittels Säulenchromatographie an 1,5 kg Silicagel in 4 Stereoisomere (A,B, C und D) der Titelverbindung aufgetrennt wird. Alle Isomeren ergeben ein analoges Massenspektrum (FAB, negativ): $(M - H)^-$= 875 (Molekulargewicht berechnet für $C_{47}H_{64}N_4O_{12}$ = 876).

Die Isomeren sind durch folgende Daten charakterisiert; die Dünnschichtchromatographie erfolgt mit Methylenchlorid-Methanol (9:1) als Lösungsmittelsystem.

| | Smp. | Rf |
|---|---|---|
| Isomeres A | 176-182° (Zersetzung) | 0,44 |
| B | 172-178° (Zersetzung) | 0,41 |
| C | 155-190° (Zersetzung) | 0,35 |
| D | 170-190° (Zersetzung) | 0,32 |

Natrium-Salz des Isomeren B wird gebildet, indem man eine Lösung von 2,5 g Isomerem B mit einer wässrigen Lösung von 0,24 g Natriumhydrogencarbonat versetzt und die Lösung lyophilisiert.

Zur Herstellung vom als Ausgangsstoff verwendeten 3-Amino-2-methyl-perhydro-1H-pyrido[1,2-a]pyrazin kann man beispielsweise 2-Methyl-perhydro-1H-pyrido[1,2-a]pyrazin, vgl. C.Winterfeld und G.Gierenz: Chem. Ber. 92, 240 (1959), gemäss Beispiel 1, Präparation a) und b) nacheinander nitrosieren und reduzieren.

Beispiel 6: Hydrazon des 3-Formylrifamycin SV mit 3-Amino-perhydro-1H-pyrazino[1,2-a]chinolin.

Eine Lösung von 5 g 3-Formylrifamycin SV in 150 ml Tetrahydrofuran wird mit einer Lösung von 3,48 g 3-Amino-perhydro-1H-pyrazino[1,2-a] chinolin in 10 ml Tetrahydrofuran versetzt und bei Raumtemperatur während 5 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert ein rohes Gemisch von 4 stereoisomeren Hydrazonen, aus dem man durch Säulenchromatographie an 200 g Silicagel 2 Stereoisomere (A und B) der Titelverbindung in reiner Form isoliert. Beide Isomeren ergeben ein analoges Massenspektrum (FAB, negativ): $(M - H)^- = 915$ (Molekulargewicht berechnet für $C_{50}H_{68}N_4O_{12} = 916$).

Isomeres A: Smp. 165-170°C (Zersetzung); Rf = 0,5

Isomeres B: Smp. 168-180°C (Zersetzung); Rf = 0,42

[Die Dünnschichtchromatographie erfolgt mit Methylenchlorid-Methanol (9:1) als Lösungsmittelsystem.]

Zur Herstellung vom als Ausgangsstoff verwendeten 3-Amino-perhydro-1H-pyrazino[1,2-a]chinolin kann man beispielsweise Perhydro-1H-pyrazino[1,2-a]chinolin gemäss Beispiel 1, Präparation a) und b) nacheinander nitrosieren und reduzieren. Die letztgenannte Verbindung ist aus 2,3,4,4a,5,6-Hexahydro-1H-pyrazino[1,2-a]chinolin, vgl. die Schweizerische Patentschrift Nr. 498,849, durch katalytisches Hydrieren an Platinoxid in Eisessig erhältlich.

Beispiel 7: Hydrazon des 3-Formylrifamycin SV mit 3-Amino-2,3,4,4a,5,6-hexahydro-1H-pyrazino[1,2-a]chinolin.

Eine Lösung von 2,5 g 3-Formylrifamycin SV in 50 ml Tetrahydrofuran wird mit einer Lösung von 0,57 g 3-Amino-2,3,4,4a,5,6-hexahydro-1H-pyrazino[1,2-a]chinolin in 10 ml Tetrahydrofuran versetzt und bei Raumtemperatur während 10 Minuten gerührt, bis gemäss Dünnschichtchromatographie alles 3-Formylrifamycin SV verbraucht wird. Zur aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Kristallisation aus Ether-Hexan die kristalline Titelverbindung ergibt; Smp. 173-180°C (Zersetzung); Dünnschichtchromatographie mit Methylenchlorid-Methanol (9:1): Rf = 0,60. Massenspektrum (DCI/$CH_4$, negativ): $M^{-\bullet}$ = 910 (Molekulargewicht berechnet für $C_{50}H_{62}N_4O_{12}$ = 910.

Zur Herstellung vom als Ausgangsstoff verwendeten 3-Amino-2,3,4,4a,5,6-hexahydro-1H-pyrazino[1,2-a]chinolin kann man beispielsweise 2,3,4,4a,5,6-Hexahydro-1H-pyrazino[1,2-a]chinolin, vgl. die Schweizerische Patentschrift Nr. 498,849 gemäss Beispiel 1, Präparation a) und b) nacheinander nitrosieren und reduzieren.

Beispiel 8: Hydrazon des 3-Formylrifamycin SV mit 6-Amino-perhydro--pyrazolo[2,1-c]-p-thiazin.

Eine Lösung von 3 g 3-Formylrifamycin SV in 50 ml Tetrahydrofuran wird mit einer Lösung von 1,0 g 6-Amino-perhydro-pyrazolo[2,1-c]-p-thiazin in 10 ml Tetrahydrofuran versetzt und bei Raumtemperatur während 10 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Kristallisation aus Aceton-Ether-Hexan kristalline Titelverbindung, Smp. 173-181°C (Zersetzung); Rf = 0,71 [Dünnschichtchromatographie mit Methylenchlorid-Methanol (9:1)] ergibt. Massenspektrum (FAB, negativ): $(M - H)^- = 879$ (Molekulargewicht berechnet für $C_{45}H_{60}N_4O_{12}S$ = 880).

Zur Herstellung vom als Ausgangsstoff verwendeten 6-Amino-perhydro--pyrazolo[2,1-c]-p-thiazin kann man beispielsweise Perhydro-pyrazolo-[2,1-c]-p-thiazin gemäss Beispiel 1, Präparation a) und b) nacheinander nitrosieren und reduzieren. Die letztgenannte Verbindung ist aus Thiomorpholin-2-carbonsäure-ethylester gemäss dem Verfahren von Freed and Day, J. Org. Chem. 25, 2108 (1960) erhältlich.

Beispiel 9: Hydrazon des 3-Formylrifamycin SV mit 3-Amino-6-methyl--perhydro-1H-pyrazino[1,2-a]pyrazin.

Eine Lösung von 3 g 3-Formylrifamycin SV in 100 ml Tetrahydrofuran wird mit einer Lösung von 2,04 g 3-Amino-6-methyl-perhydro-1H-pyrazino[1,2-a]pyrazin in 10 ml Tetrahydrofuran versetzt und bei Raumtemperatur während 15 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Kristallisation aus Aceton-Ether ein kristallines Gemisch von 2 Stereoisomeren der Titelverbindung ergibt, welches Smp. 170°C (Zersetzung) aufweist und in Dünnschichtchromatographie mit Methylenchlorid-Methanol (9:1) 2 Produkte vom $Rf_A = 0{,}47$ und $Rf_B = 0{,}41$ zum Vorschein bringt. Massenspektrum (FAB, negativ):$(M - H)^- = 876$ (Molekulargewicht berechnet für $C_{46}H_{63}N_5O_{12} = 877$).

Zur Herstellung vom als Ausgangsstoff verwendeten 3-Amino-6-methyl--perhydro-1H-pyrazino[1,2-a]pyrazin kann man beispielsweise 6-Methyl--perhydro-pyrazino[1,2-a]pyrazin, vgl. H.J. Beim und A.R. Day: J. Heterocyclic Chem. 14, 307 (1977), gemäss Beispiel 1, Präparation a) und b) nacheinander nitrosieren und reduzieren.

Beispiel 10: Hydrazon des 3-Formylrifamycin SV mit 3-Amino-6-isobutyl--perhydro-1H-pyrazino[1,2-a]pyrazin.

Eine Lösung von 3 g 3-Formylrifamycin SV in 100 ml Tetrahydrofuran wird mit einer Lösung von 1,73 g 3-Amino-6-isobutyl-perhydro-1H-pyrazino[1,2-a]pyrazin in 10 ml Tetrahydrofuran versetzt und bei Raumtemperatur während 60 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Säulenchromatographie an 200 g Silicagel gereinigte Titelverbindung, Smp. 163-170°C

(Zersetzung): Rf = 0,42 [Dünnschichtchromatographie in Methylenchlorid-Methanol (9:1)] ergibt. Massenspektrum (FAB, negativ): $(M - H)^{-}$ = 918 (Molekulargewicht berechnet für $C_{49}H_{69}N_5O_{12}$ = 919).

Zur Herstellung vom als Ausgangsstoff verwendeten 3-Amino-6-isobutyl--perhydro-1H-pyrazino[1,2-a]pyrazin kann man beispielsweise 6-Isobutyl--perhydro-1H-pyrazino[1,2-a]pyrazin, hergestellt nach H.J. Beim und A.R. Day: J. Heterocyclic Chem. 14, 307 (1977), gemäss Beispiel 1, Präparation a) und b) nacheinander nitrosieren und reduzieren.

Beispiel 11: Hydrazon des 3-Formylrifamycin SV mit 3-Amino-6-cyclopentyl-perhydro-1H-pyrazino[1,2-a]pyrazin.

Eine Lösung von 3 g 3-Formylrifamycin SV in 50 ml Tetrahydrofuran wird mit einer Lösung von 1,8 g 3-Amino-6-cyclopentyl-perhydro-1H-pyrazino-[1,2-a]pyrazin in 10 ml Tetrahydrofuran versetzt und bei Raumtemperatur während 15 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Chromatographie an 200 g Silicagel die Titelverbindung ergibt. Massenspektrum (FAB, negativ): $(M - H)^{-}$ = 930 (Molekulargewicht berechnet für $C_{50}H_{69}N_5O_{12}$ = 931).

Zur Herstellung vom als Ausgangsstoff verwendeten 3-Amino-6-cyclopentyl-perhydro-1H-pyrazino[1,2-a]pyrazin kann man beispielsweise 6-Cyclopentyl-perhydro-1H-pyrazino[1,2-a]pyrazin, hergestellt nach H.J. Beim und A.R. Day: J. Heterocyclic Chem. 14, 307 (1977), gemäss Beispiel 1, Präparation a) und b) nacheinander nitrosieren und reduzieren.

Beispiel 12: Hydrazon des 3-Formylrifamycin SV mit 6-Amino-perhydro--pyrazino[2,1-c]-p-oxazin.

Eine Lösung von 3 g 3-Formylrifamycin SV in 100 ml Tetrahydrofuran wird mit einer Lösung von 1,94 g 6-Amino-perhydro-pyrazino[2,1-c]-p--oxazin in 10 ml Tetrahydrofuran versetzt und bei Raumtemperatur während 10 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter wässriger Zitronensäure-Lösung gewaschen, getrocknet und eingedampft. Es resultiert rohes Hydrazon, welches durch Kristallisation aus Ethylacetat-Cyclohexan orangerote Kristalle der Titelverbindung ergibt. Massenspektrum (FAB, negativ):$(M - H)^{-}$ = 863 (Molekulargewicht berechnet für $C_{45}H_{60}N_4O_{13}$ = 864).

Zur Herstellung vom als Ausgangsstoff verwendeten 6-Amino-perhydro--pyrazino[2,1-c]-p-oxazin kann man beispielsweise Perhydro-pyrazino-[2,1-c]-p-oxazin gemäss Beispiel 1, Präparation a) und b) nacheinander nitrosieren und reduzieren. Die letztgenannte Verbindung ist aus Morpholino-2-carbonsäure-ethylester gemäss dem Verfahren von Freed and Day, J.Org. Chem. 25, 2108 (1960) erhältlich.

Beispiel 13: Hydrazon von 3-Formylrifamycin SV und 3-Amino-7-methoxy--perhydro-1H-pyrido[1,2-a]pyrazin.

Eine Lösung von 3 g 3-Formylrifamycin SV in 100 ml Tetrahydrofuran wird mit einer Lösung von 1,37 g 3-Amino-7-methoxy-perhydro-1H-pyrido-[1,2-a]pyrazin in 10 ml Tetrahydrofuran versetzt und bei Raumtemperatur während 60 Minuten gerührt, bis gemäss Dünnschichtchromatographie alles 3-Formylrifamycin SV verbraucht wird. Zur Aufarbeitung wird die Reaktionslösung im Vakuum eingeengt und der Rückstand in Methylenchlorid aufgenommen. Die organische Phase wird zweimal mit verdünnter

wässriger Zitronensäure-Lösung gewaschen, getrocknet und eingedampft. Es resultiert rohes Hydrazon, welches durch Säulenchromatographie an 200 g Silicagel gereinigte Titelverbindung ergibt. Massenspektrum (FAB, negativ): $(M - H)^- = 891$ (Molekulargewicht berechnet für $C_{47}H_{64}N_4O_{13} = 892$).

Zur Herstellung vom als Ausgangsstoff verwendeten 3-Amino-7-methoxy-perhydro-1H-pyrido[1,2-a]pyrazin kann man beispielsweise 7-Methoxy--perhydro-1H-pyrido[1,2-a]pyrazin gemäss Beispiel 1, Präparation a) und b) nacheinander nitrosieren und reduzieren. Die letztgenannte Verbindung ist aus 2-Methoxy-6-piperidin-carbonsäure-ethylester gemäss dem Verfahren von Freed and Day, J. Org. Chem. 25, 2108 (1960) erhältlich.

Beispiel 14: Kapseln, enthaltend 250 mg Hydrazon des 3-Formylrifamycin SV mit 3-Amino-perhydro-1H-pyrido[1,2-a]pyrazin, können wie folgt hergestellt werden:

Zusammensetzung (für 1000 Kapseln):

| | |
|---|---|
| Hydrazon des 3-Formylrifamycin SV mit 3-Amino-perhydro-1H-pyrido[1,2-a]pyrazin | 250,0 g |
| Maisstärke | 50,0 g |
| Polyvinylpyrrolidon | 15,0 g |
| Magnesiumstearat | 5,0 g |
| Ethanol | q.s. |

Der Wirkstoff und die Maisstärke werden vermischt und mit einer Lösung des Polyvinylpyrrolidons in 50 g Ethanol befeuchtet. Die feuchte Masse wird durch ein Sieb mit einer Maschenweite von 3 mm gepresst und bei 45°C getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse 0 abgefüllt.

In analoger Weise werden Kapseln enthaltend eine äquivalent wirksame Dosis jedes einzelnen Produkts der Beispiele 2-13 hergestellt.

0135837

Patentansprüche:

1. Eine Verbindung der Formel

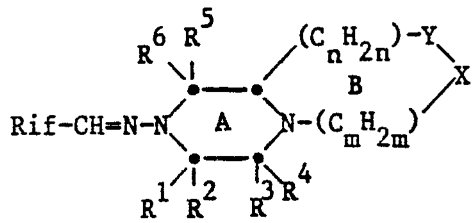

(I)

worin $R^1$, $R^2$, $R^3$, $R^4$, $R^5$ und $R^6$ unabhängig je ein Wasserstoffatom oder $C_{1-4}$-Alkyl,

m und n unabhängig je eine ganze Zahl von 0 bis 5,

X $C_{1-5}$-Alkyliden, Benzyliden oder $C_{1-4}$-Alkoxymethylen,

Y $C_{1-5}$-Alkyliden, $C_{1-4}$-Alkoxymethylen, Oxy, Thio oder gegebenenfalls substituiertes Imino der Formel -N(R)-, worin R für Wasserstoff, $C_{1-4}$-Alkyl, $C_{3-5}$-Alkenyl, $C_{3-12}$-Cycloalkyl oder Phenyl steht, oder

X und Y zusammen ein 1,2-Cycloalkylen oder o-Phenylen, welche durch 1-3 $C_{1-4}$-Alkylreste substituiert sein können, und

Rif einen Rest der Partialformeln

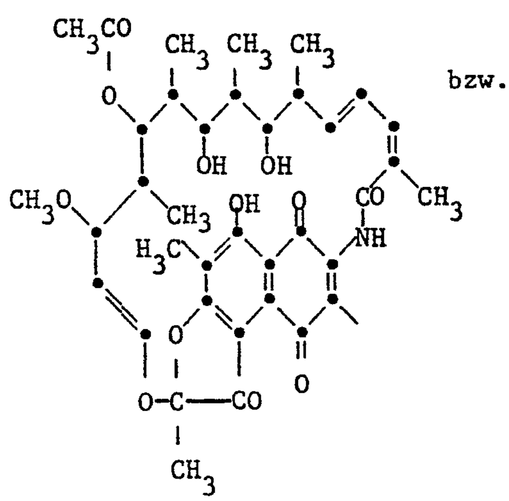

[Rif S]

bzw.

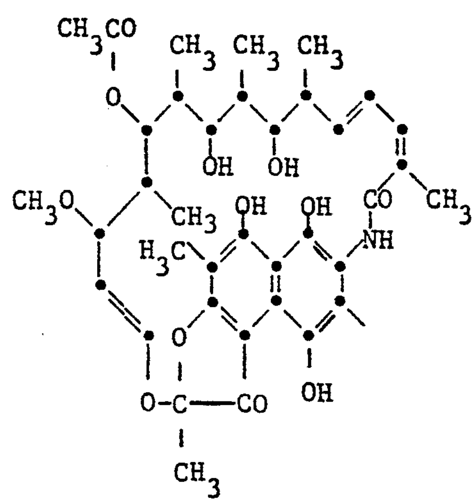

[Rif SV]

darstellt, und Salze davon.

2. Eine Verbindung gemäss Anspruch 1, worin m gleich 0 bis 3 und n gleich 1 bis 4 ist, wobei jeder der Reste $C_mH_{2m}$ und $C_nH_{2n}$ den Piperazinring A vom Symbol X bzw. Y durch höchstens 3 C-Atome trennt, höchstens einer der Symbole $R^1$-$R^6$ von Wasserstoff unterschiedlich ist und Methyl oder Ethyl bedeutet, und eines der Symbole X und Y Ethyliden oder Methylen, während das andere $C_{1-4}$-Alkyliden, $C_{1-4}$-Alkoxymethylen, Oxy (-O-), Thio (-S-), Imino (-NH-) oder durch $C_{1-4}$-Alkyl substituiertes Imino darstellt oder X und Y zusammen für ein 1,2-Cycloalkylen mit 6-8 Ringatomen oder o-Phenylen stehen.

3. Eine Verbindung gemäss Anspruch 1, worin m gleich 0 und n in einem geradkettigen Rest $C_nH_{2n}$ gleich 1 bis 4 ist, höchstens eines der Symbole $R^1$-$R^6$ von Wasserstoff unterschiedlich ist, in welchem Falle es Methyl bedeutet, und eines der Symbole X und Y Methylen, während das andere Ethyliden, Propyliden, $C_{1-4}$-Alkoxymethylen oder Methylen darstellt, oder X und Y zusammen für 1,2-Cyclohexylen oder o-Phenylen stehen.

4. Eine Verbindung gemäss Anspruch 1, worin je m und n unabhängig gleich 1 bis 3 ist, wobei die Reste $C_mH_{2m}$ und $C_nH_{2n}$ den Piperazinring A vom Symbol X bzw. Y jeweils durch 1 Kohlenstoffatom trennen, höchstens eines der Symbole $R^1$-$R^6$ von Wasserstoff unterschiedlich ist, in welchem Falle es Methyl bedeutet, und eines der Symbole X und Y Ethyliden oder Methylen, während das andere Oxy, Thio oder Imino darstellt, wobei das Imino durch $C_{1-4}$-Alkyl, $C_{5-8}$-Cycloalkyl oder Phenyl substituiert sein kann.

5. Eine Verbindung gemäss Anspruch 1, die das Hydrazon des 3-Formylrifamycin SV oder S mit 3-Amino-perhydro-1H-pyrido[1,2-a]pyrazin ist.

6. Eine Verbindung gemäss Anspruch 1, die das Hydrazon des 3-Formylrifamycin SV oder S mit 3-Amino-perhydro-pyrrolo[1,2-a]pyrazin ist.

7. Eine Verbindung gemäss Anspruch 1, die das Hydrazon des 3-Formylrifamycin SV oder S mit 3-Amino-8-methyl-perhydro-1H-pyrido[1,2-a]pyrazin ist.

8. Eine Verbindung gemäss Anspruch 1, die das Hydrazon des 3-Formylrifamycin SV oder S mit 3-Amino-7-ethyl-perhydro-1H-pyrido[1,2-a]pyrazin ist.

9. Eine Verbindung gemäss Anspruch 1, die das Hydrazon des 3-Formylrifamycin SV oder S mit 3-Amino-2-methyl-perhydro-1H-pyrido[1,2-a]pyrazin ist.

10. Eine Verbindung gemäss Anspruch 1, die das Hydrazon des 3-Formylrifamycin SV oder S mit 3-Amino-perhydro-1H-pyrazino[1,2-a]chinolin ist.

11. Eine Verbindung gemäss Anspruch 1, die das Hydrazon des 3-Formylrifamycin SV oder S mit 3-Amino-2,3,4,4a,5,6-hexahydro-1H-pyrazino-[1,2-a]chinolin ist.

12. Eine Verbindung gemäss Anspruch 1, die das Hydrazon des 3-Formylrifamycin SV oder S mit 6-Amino-perhydro-pyrazolo[2,1-c]-p-thiazin ist.

13. Eine Verbindung gemäss Anspruch 1, die das Hydrazon des 3-Formylrifamycin SV oder S mit 3-Amino-6-R-perhydro-1H-pyrazino[1,2-a]pyrazin, worin R Methyl, Isobutyl oder Cyclopentyl bedeutet, ist.

14. Eine Verbindung gemäss Anspruch 1, die das Hydrazon des 3-Formylrifamycin SV oder S mit 6-Amino-perhydro-pyrazolo[2,1-c]-p-oxazin ist.

15. Eine Verbindung gemäss Anspruch 1, die das Hydrazon des 3-Formylrifamycin SV oder S mit 3-Amino-7-methoxy-perhydro-1H-pyrido[1,2-a]pyrazin ist.

16. Eine Verbindung gemäss einem der Ansprüche 1-15, die sich vom Rifamycin SV ableitet, oder ein pharmazeutisch anwendbares Alkalimetallsalz davon.

17. Eine im Anspruch 1 definierte Verbindung der Formel I oder ein pharmazeutisch verwendbares Salz davon zur Verwendung als Arzneimittel.

18. Eine im Anspruch 1 definierte Verbindung der Formel I oder ein pharmazeutisch verwendbares Salz davon mit antituberkulösen und/oder antibakteriellen Eigenschaften.

19. Verfahren zur Herstellung von einer im Anspruch 1 definierten Verbindung der Formel I oder eines Salzes davon, dadurch gekennzeichnet, dass man

a) ein 3-Formylrifamycin der Formel

$$Rif - CH = O \qquad (II)$$

worin Rif die genannte Bedeutung hat, oder ein funktionelles Derivat davon, mit einem N-Aminopiperazin der Formel

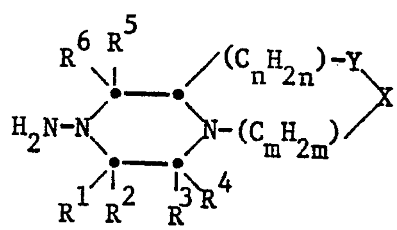

(III)

worin $R^1$-$R^6$, n, m, X und Y die im Anspruch 1 angegebenen Bedeutungen haben, umsetzt, oder

b) zur direkten Herstellung von Derivaten der S-Reihe die 3-(Rifamycin S)-sulfonsäure der Formel

$$[Rif\ S]-SO_3H \qquad (IV)$$

worin [Rif S] der im Anspruch 1 angegebenen Formel entspricht, mit Formaldehydhydrazon eines N-Aminopiperazins der Formel

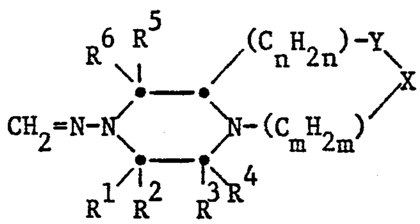

(V)

worin $R^1$-$R^6$, n, m, X und Y die im Anspruch 1 angegebenen Bedeutungen haben, umsetzt, und,

gewünschtenfalls, wenn eine Verbindung der Formel I in der Chinon-Form erwünscht ist, eine in der Hydrochinon-Form vorliegende Verbindung der Formel I mit einem Oxidationsmittel behandelt, und/oder, wenn eine Verbindung der Formel I in der Hydrochinon-Form erwünscht ist, eine in der Chinon-Form vorliegende Verbindung der Formel I mit einem Reduktionsmittel behandelt und/oder eine in freier Form vorliegende Verbindung der Formel I in ein Salz davon überführt oder die Verbindung der Formel I aus einem Salz davon freisetzt.

20. Verfahren gemäss Anspruch 19, dadurch gekennzeichnet, dass man eine in einem der Ansprüche 2-16 definierte Verbindung herstellt.

21. Pharmazeutische Zusammensetzung enthaltend eine im Anspruch 1 definierte Verbindung der Formel I oder ein pharmazeutisch verwendbares Salz davon.

22. Pharmazeutische Zusammensetzung gemäss Anspruch 21 enthaltend eine in einem der Ansprüche 2-18 definierte Verbindung der Formel I oder

ein pharmazeutisch verwendbares Salz davon.

23. Verwendung einer der im Anspruch 1 definierten Verbindungen der Formel I oder eines pharmakologisch verwendbaren Salzes davon zur Herstellung von pharmazeutischen Zusammensetzungen gemäss Anspruch 21 oder 22.

24. Verfahren zur Herstellung von pharmazeutischen Zusammensetzungen enthaltend als Wirkstoffe eine in einem der Ansprüche 1-18 definierte Verbindung der Formel I oder ein pharmakologisch verwendbares Salz davon, gekennzeichnet durch Vermischen dieses Wirkstoffs mit mindestens einem pharmazeutischen Träger- und/oder Hilfsmaterial auf nicht-chemischem Wege.

25. Verwendung einer der im Anspruch 1 definierten Verbindungen der Formel I oder eines pharmakologisch verwendbaren Salzes davon allein oder in Form einer pharmazeutischen Zusammensetzung gemäss Anspruch 20 oder 21 zur Behandlung von Infektionen des tierischen oder menschlichen Körpers.

26. Methode zur Behandlung von Infektionen des tierischen oder menschlichen Körpers, gekennzeichnet durch die Verabreichung einer antimikrobiell wirksamen Dosis einer der im Anspruch 1 definierten Verbindungen der Formel I oder eines pharmakologisch verwendbaren Salzes davon allein oder in Form einer pharmazeutischen Zusammensetzung gemäss Anspruch 21 oder 22 an das Subjekt in Bedürfnis davon.